# EUROPÄISCHE PATENTSCHRIFT

(11) **EP 2 544 743 B1**
(45) Veröffentlichungstag und Bekanntmachung des Hinweises auf die Patenterteilung: **22.01.2014**
(21) Anmeldenummer: 11719444.9
(22) Anmeldetag: 10.03.2011
(51) Int. Cl.: A61M 15/00, A61M 11/04, A24F 47/00

(54) **FLÄCHIGER VERDAMPFER**
PLANAR EVAPORATOR
EVAPORATEUR PLAN

(30) Priorität: 10.03.2010 AT 3862010
(43) Veröffentlichungstag der Anmeldung: 16.01.2013
(73) Patentinhaber: Batmark Limited, London WC2R 2PG (GB)
(72) Erfinder: BUCHBERGER, Helmut, A-4482 Ennsdorf (AT)
(74) Vertreter: Schrell, Andreas
(86) Internationale Anmeldenummer: PCT/AT2011/000123
(87) Internationale Veröffentlichungsnummer: WO 2011/109849

(56) Entgegenhaltungen:
- US-A- 4 588 976
- US-A- 5 179 966
- US-A- 5 322 075
- US-A1- 2003 049 025
- US-A1- 2007 155 255

## Beschreibung

Die Erfindung betrifft einen flächigen Verdampfer, umfassend ein flächiges elektrisches Widerstandsheizelement zur impulsartigen Aufheizung und Verdampfung eines auf der Heizelementfläche verteilten oder verteilbaren und inhalativ aufnehmbaren Stoffes mittels eines flächig durch das Widerstandsheizelement fließenden oder fließbaren elektrischen Heizstromes mit zumindest zwei elektrischen Kontaktierungen bzw. Polen zur Einleitung des Heizstromes in das Widerstandsheizelement.

### Begriffsdefinition:

Der Begriff "inhalativ aufnehmbarer Stoff" umfaßt grundsätzlich jeden von einem Menschen bzw. Benutzer inhalierbaren Stoff. Der verdampfte Stoff kann dem Benutzer in Form eines Dampf-Luft-Gemisches oder/und Kondensationsaerosols zugeführt werden. Der Stoff kann ein Arzneimittel enthalten, oder aus einer Arzneimittel-Zubereitung bestehen; er kann aber auch nur Bestandteile enthalten, welche nicht als Arzneimittel deklariert sind.

Eine "impulsartigen Aufheizung und Verdampfung" liegt vor, wenn der Stoff innerhalb der Zeitspanne einer Inhalation, also innerhalb weniger Sekunden oder innerhalb von Bruchteilen einer Sekunde aufgeheizt und verdampft wird.

Der "flächig durch das Widerstandsheizelement fließende oder fließbare elektrische Heizstrom" kann ein Gleichstrom oder Wechselstrom sein.

Bekannte flächige Verdampfer, wie sie beispielsweise in US 2007/155255 (Charles Galauner et al.), US 4,735,217 (Donald L. Gerth et al.), US 2005/0268911 (Steven D. Cross et al.), US 5,060,671 (Mary E. Counts et al.), US 5,095,921 (D. Bruce Losee et al.) und US 4,922,901, Fig. 4-8 (Johnny L. Brooks et al.) beschrieben werden, weisen in weiten Bereichen der Verdampfungsoberfläche weitgehend konstante Temperaturverhältnisse auf - vgl. zum Beispiel US 2005/0268911, Fig. 17a und 17b. Größere Temperaturgradienten treten allenfalls im Bereich der elektrischen Kontaktierung auf, wo der elektrische Strom in das flächige Widerstandsheizelement eingeleitet wird, und Wärme parasitär in angrenzende Strukturelemente abfließt. Ansonsten ist die Temperaturverteilung jedoch sehr gleichmäßig. Diese gleichmäßige Temperaturverteilung kann insbesondere dann von Nachteil sein, wenn der zu verdampfende Stoff Bestandteile mit unterschiedlichen Siedepunkten beinhaltet. Die flächig konstante Temperaturverteilung hat in diesem Fall nämlich den Effekt, daß der Stoff mit dem niedrigeren Siedepunkt im Zuge der impulsartigen Energiezufuhr zuerst verdampft, und der Stoff mit dem höheren Siedepunkt erst zu verdampfen beginnt, wenn der niedriger siedende Stoff bereits weitgehend verdampft ist und die Verdampfungszone bereits verlassen hat. Konkret wirkt sich dieser Effekt dann nachteilig aus, wenn die beiden Stoffe unmittelbar nach ihrer Verdampfung bzw. Freisetzung in einer bestimmten beabsichtigten Weise miteinander wechselwirken sollen, um beispielsweise bestimmte pharmakologische oder/ und pharmakokinetische Wirkungen zu entfalten, oder solche Wirkungen zu begünstigen. Ein Beispiel für solche Wechselwirkungen ist die Anlagerung eines ansonsten flüchtigen Stoffes an eine aerosolbildende Substanz. Aerosolbildende Substanzen zeichnen sich durch besonders geringe Dampfdrucke aus. Als Beispiel für eine aerosolbildende Substanz sei Glycerol (Glyzerin) genannt. Aerosolbildende Substanzen haben die Aufgabe, ansonsten flüchtige Stoffe an sich zu binden, und auf diese Weise ihre Lungengängigkeit zu verbessern, um so beispielsweise eine systemische Wirkstoffzufuhr zu ermöglichen. Ein weiteres Beispiel für eine solche Wechselwirkung ist die Protonierung von Nikotin durch Säuren bzw. die Bildung von entsprechenden Nikotinsalzen. Nikotin verdampft im Wesentlichen als freie Base. Freies Nikotin in Basenform ist jedoch leicht flüchtig und in dieser Form kaum lungengängig. Der Großteil der Nikotinbase würde bereits früher abgeschieden. Eine systemische Nikotinverabreichung über die Lungenalveolen ist auf diese Weise kaum realisierbar. Rekombiniert das Nikotin jedoch unmittelbar nach seiner Verdampfung bzw. Freisetzung mit einer Säure, kann seine Flüchtigkeit wesentlich herabgesetzt werden, insbesondere wenn gleichzeitig auch aerosolbildende Substanzen verfügbar sind. In einer glimmenden Zigarette sind diese Verhältnisse während eines Zuges in optimaler Weise erfüllt. Dies ist vor allem dem steilen Temperaturgefälle zwischen der Glutzone und der Verdampfungs- und Destillationszone zuzuschreiben. Die Temperatur fällt hier innerhalb von wenigen Millimetern von über 800°C auf unter 100°C ab. Unter diesen Bedingungen werden aerosolbildende Substanzen, organische Säuren und Nikotin auf engstem Raum praktisch synchron freigesetzt, auch wenn sich die Siedepunkte bzw. Freisetzungstemperaturen der aerosolbildenden Substanzen und organischen Säuren deutlich vom Siedepunkt des Nikotins (246°C) unterscheiden. Die Wahrscheinlichkeit, daß unter diesen Bedingungen das Nikotin mit einer Säure rekombiniert und sich an ein bereits kondensiertes Aerosolpartikel anlagert, ist entsprechend hoch. Diese Bedingungen führen bei filterlosen Zigaretten letztlich dazu, daß das in den Hauptstromrauch übergegangene Nikotin zu einem Großteil tatsächlich die Lungenalveolen erreicht und die gewünschte systemische Wirkung im Zentralnervensystem innerhalb von Sekunden entfalten kann.

Der Erfindung liegt die Aufgabe zugrunde, die zuvor aufgezeigten Nachteile der aus dem Stand der Technik bekannten Anordnungen flächiger Verdampfer zu beheben. Der Erfindung liegt insbesondere die Aufgabe zugrunde, einen flächigen Verdampfer der eingangs geschilderten Art so auszugestalten, daß im zu verdampfenden Stoff während der impulsartigen Stromzufuhr in Flächenrichtung und auf engstem Raum ein möglichst steiles Temperaturgefälle bzw. ein möglichst großer Temperaturgradient auftritt, sodaß die im Stoff enthaltenen Einzelsubstanzen möglichst synchron freigesetzt werden.

Die Aufgabe wird dadurch gelöst, daß das Widerstandsheizelement mindestens eine die Feldlinien des sich zwischen den Polen ausbildenden bzw. ausbildbaren elektrischen Ursprungsfeldes einschnürende schlitzförmige Ausnehmung aufweist und flächig mit einer den Stoff aufnehmenden oder aufnehmbaren offenzelligen Porenstruktur verbunden ist.

Begriffsdefinition: Das "elektrische Ursprungsfeld" ist jenes elektrische Feld, welches sich im Widerstandsheizelement ausbildete, wenn keine erfindungsgemäße schlitzförmige Ausnehmung vorhanden wäre.

Die offenzellige Porenstruktur kann beispielsweise durch ein Gewebe, eine offenporige Faserstruktur, eine offenporige Sinterstruktur, einen offenporigen Schaum oder durch eine offenporige Abscheidungsstruktur gebildet werden. Auch eine Kombination dieser Strukturen ist möglich. Die Strukturen können ferner in mehreren Lagen übereinander geschichtet sein.

In Analogie zu einem fließenden Gewässer wirkt die schlitzförmige Ausnehmung wie ein sich quer zum Gewässerlauf erstreckender Steg: es bilden sich im Bereich um die schlitzförmige Ausnehmung sowohl Zonen mit einem erhöhten als auch Zonen mit einem verminderten Stromfluss aus. Der Umstand, daß die elektrische Leistungsdichte proportional zum Quadrat der Stromdichte ist, führt dazu, daß die in das Widerstandsheizelement eingebrachte Wärme im Bereich um die schlitzförmige Ausnehmung von einem Punkt zum anderen stark fluktuiert, und sich in Flächenrichtung steile Temperaturgradienten ausbilden. Das Temperaturgefälle kann auf engstem Raum, das heißt innerhalb von Distanzen, welche der Ausdehnung der schlitzförmigen Ausnehmung entsprechen, realisiert werden. Die eingebrachte Wärme wird durch Wärmeleitung auf den in der unmittelbar angrenzenden Porenstruktur gespeicherten Stoff übertragen. Hierbei treten zwei Eigenschaften der Porenstruktur in Erscheinung, welche für die gegenständliche Erfindung maßgeblich sind: zum einen die Eigenschaft, daß die Wärmeleitfähigkeit in Porenstrukturen mit zunehmender Porosität überproportional abnimmt; zum anderen die Eigenschaft, daß Porenstrukturen auch vergleichsweise große Stoffmengen aufnehmen und fixieren können. Die Porenstruktur wirkt also sowohl einem Wärmeaustausch als auch einem Stoffaustausch in Flächenrichtung entgegen. Diese Eigenschaften haben den Effekt, daß die im Widerstandsheizelement ausgebildeten Temperaturgradienten von der Porenstruktur kaum tangiert werden, und dieselbigen Temperaturgradienten sich auch in der Porenstruktur und letztlich im zu verdampfenden Stoff in vergleichbarer Größe ausbilden können. Als vorteilhaft kann schließlich auch noch gewertet werden, daß durch die schlitzförmige Ausnehmung die Wärmeverluste des Widerstandsheizelementes insgesamt nicht erhöht werden.

In einer vorteilhaften Weiterbildung der Erfindung ist vorgesehen, daß die Porenstruktur selbst aus einem elektrischen Widerstandsmaterial besteht, und die schlitzförmige Ausnehmung auch die Porenstruktur durchsetzt. Die Porenstruktur wird also selbst ein Teil des Widerstandsheizelementes. Diese Anordnung hat den vorteilhaften Effekt, daß die Wärme zumindest teilweise direkt in der Porenstruktur erzeugt wird und dort unmittelbar auf den zu verdampfenden Stoff übertragen wird. Wie bereits erwähnt wurde, nimmt die Wärmeleitfähigkeit in Porenstrukturen mit zunehmender Porosität überproportional ab. So günstig sich diese Eigenschaft im Hinblick auf die Ausbildung von Temperaturgradienten in Flächenrichtung erweist, so nachteilig ist diese Eigenschaft zu werten, wenn daraus auch ein **Temperaturgradient in Dickenrichtung resultiert. Ein Temperaturgradient in** Dickenrichtung kann nämlich die Verdampfung und damit die Leistungsfähigkeit des Verdampfers empfindlich stören, insofern, als dadurch das Auftreten einer Siedekrise begünstigt wird. Dies gilt vor allem in solchen Fällen, wo die Porenstrukur weitgehend oder vollständig mit dem zu verdampfenden Stoff gesättigt ist. Dadurch, daß die Wärme zumindest teilweise direkt in der Porenstruktur erzeugt wird, kann der Ausbildung eines Temperaturgradienten in Dickenrichtung effektiv entgegengewirkt werden. In einem erfindungsgemäßen Spezialfall kann vorgesehen sein, daß das Widerstandsheizelement komplett von der Porenstruktur ausgebildet wird. Die Wärme wird in diesem Fall komplett in der Porenstruktur erzeugt. Solche Anordnungen machen naturgemäß die höchsten Verdampfungsleistungen realisierbar, vor allem, wenn die Poren der Porenstruktur auf beiden Seiten freiliegen, das heißt mit der Umgebung frei kommunizieren, und der Dampf folglich beidseitig aus dem flächigen Heizelement ausströmen kann. Die Poren der Porenstruktur gelten übrigens nicht als Ausnehmungen im Sinne der vorliegenden Anmeldung, auch dann nicht, wenn sie schlitzförmig sind. Eine schlitzförmige Ausnehmung gelte nur dann als erfindungsgemäß, wenn sie sich zumindest über mehrere Poren hinweg erstreckt.

Besonders günstig ist es, wenn die schlitzförmige Ausnehmung im Wesentlichen geradlinig verläuft und zu den von ihr eingeschnürten Feldlinien des elektrischen Ursprungsfeldes zumindest annähernd orthogonal ausgerichtet ist. Für die Beurteilung, ob die zuvor genannten Verhältnisse gegeben sind oder nicht, sind jene ungestörten Feldlinien des elektrischen Ursprungsfeldes geometrisch auszuwerten, welche von der schlitzförmigen Ausnehmung am stärksten eingeschnürt werden. Es kann gezeigt werden, daß schlitzförmige Ausnehmungen vorgegebener Größe den größten Einschnüreffekt haben, wenn sie die zuvor genannten Geometriebedingungen erfüllen.

Gemäß einer bevorzugten Ausführungsform der Erfindung besteht die schlitzförmige Ausnehmung aus einem Einschnitt. Der Einschnitt wird vorzugsweise mittels Laserschneidverfahren hergestellt. Laserschneidverfahren erlauben die Herstellung von besonders feinen Schlitzen. So können mittels Laserfeinschnitt-Verfahren, beispielsweise mittels Nd:YAG-Laser, maß- und formhaltige Schlitze bzw. Einschnitte mit einer Breite ab etwa 50µm gesetzt werden. Solch feine Einschnitte gestatten es, die Feldlinien des elektrischen **Ursprungsfeldes** einzuschnüren, **ohne hierfür eine nennenswerte Fläche zu** beanspruchen, so daß - geometrisch betrachtet - beinahe die gesamte ursprüngliche Heizelementfläche für die Verdampfung verfügbar bleibt.

In einer weiteren günstigen Ausgestaltung der Erfindung bestehen das Widerstandsheizelement und die Porenstruktur aus einem metallischen Widerstandsmaterial. Das Laserschneiden von flächigen metallischen Werkstoffen wie Feinblechen, Metallfolien und Metallgeweben wird heute routinemäßig durchgeführt. Das Setzen eines erfindungsgemäßen Einschnittes stellt also bei solchen Werkstoffen keine große technologische Herausforderung dar, auch dann nicht, wenn der Werkstoff eine Porenstruktur aufweist. Geeignete metallische Widerstandsmaterialien sind beispielsweise: Edelstähle wie AISI 304 oder AISI 316 sowie Heizleiterlegierungen - insbesondere NiCr-Legierungen und CrFeAl-Legierungen ("Kanthal") wie DIN-Werkstoff-Nummer 2.4658, 2.4867, 2.4869, 2.4872, 1.4843, 1.4860, 1.4725, 1.4765, 1.4767. Die Erfindung ist jedoch keineswegs auf diese Metalle bzw. Legierungen beschränkt. Die angeführten Metalle weisen im Vergleich zu vielen nichtmetallischen Widerstandsmaterialien eine vergleichsweise hohe elektrische Leitfähigkeit auf. Entsprechend hohe Heizströme sind die Folge. Besonders wenn diese Ströme aus Batterien bezogen werden sollen, stößt man unter Umständen technologisch bereits an gewisse Grenzen. In diesem Zusammenhang erweist sich der erfindungsgemäße schlitzförmige Einschnitt insofern als vorteilhaft, als der Widerstand des Heizelementes durch den Einschnitt erhöht wird. Der Schlitz ermöglicht außerdem die exakte Einstellung eines vorgegebenen Soll-Widerstandswertes.

In einer bevorzugten Weiterbildung der Erfindung sind mehrere oder eine Vielzahl von schlitzförmigen Ausnehmungen vorgesehen. Durch das Vorsehen mehrerer oder gar einer Vielzahl von schlitzförmigen Ausnehmungen können auf der Heizelementfläche mehrere bzw. eine Vielzahl von Zonen mit lokal ausgeprägtem Temperaturgefälle geschaffen werden, und die bereits beschriebenen günstigen Effekte weitflächiger wirksam werden.

Nach der Erfindung können die schlitzförmigen Ausnehmungen ungleichmäßig dicht auf der Heizelementfläche verteilt sein. Durch die ungleichmäßige Verteilung der schlitzförmigen Ausnehmungen kann dem Widerstandsheizelement bzw. der Porenstruktur ein zweiter Typ von Temperaturgefälle aufgeprägt werden, welcher die Zonen mit lokal ausgeprägtem Temperaturgefälle überlagern, und dadurch weitläufiger wirkt. Allgemeiner formuliert kann durch die ungleichmäßige Verteilung der schlitzförmigen Ausnehmungen Einfluß auf die Strom- und Leistungsdichteverteilung genommen werden. So können beispielsweise Zonen hoher Stromdichte durch das Einbringen schlitzförmiger Ausnehmungen entlastet werden, da der Strom nun diese Zonen umgehen wird.

In einer besonders vorteilhaften Weiterbildung des erfindungsgemäßen flächigen Verdampfers ist vorgesehen, daß die Porenstruktur einen Docht ausbildet. Der Docht bewirkt durch seine Kapillarität, daß sich die Porenstruktur nach einer impulsartigen Verdampfung von neuem selbsttätig mit dem in diesem Fall flüssigen Stoff füllen kann. Die Porenstruktur ist zu diesem Zweck lediglich mit einer Flüssigkeitsquelle in Kontakt zu bringen. Die Porenstruktur wirkt also bei dieser Ausgestaltungsvariante multifunktionell.

Die Erfindung betrifft außerdem eine Inhalatorkomponente, umfassend einen erfindungsgemäßen flächigen Verdampfer, wie zuletzt beschrieben, sowie eine mit dem Docht kapillar kommunizierende bzw. kommunizierbare Flüssigkeitsquelle zur Versorgung des Dochts mit dem flüssigen Stoff. Erfindungsgemäß ist vorgesehen, daß der Docht in Richtung der Feldlinien des elektrischen Ursprungsfeldes mit dem flüssigen Stoff versorgt wird, und die schlitzförmigen Ausnehmungen hintereinander gestaffelt in einer zu den Feldlinien im Wesentlichen parallel ausgerichteten Reihe angeordnet sind. Die Beschickung des Dochts mit dem flüssigen Stoff in Richtung der Feldlinien des elektrischen Ursprungsfeldes kann vorteilhafterweise über einen elektrischen Kontaktstützpunkt erfolgen, wodurch zusätzliche Wärmeverluste vermieden werden können. Durch die reihenförmige Gruppierung der schlitzförmigen Ausnehmungen wird eine dem Docht vorbehaltene Zone geschaffen, welche weitgehend frei von schlitzförmigen Ausnehmungen ist, und über welche die Porenstruktur weitgehend ungehindert mit dem flüssigen Stoff versorgt werden kann. Die schlitzförmigen Ausnehmungen können auch in mehreren Reihen gestaffelt sein.

Alternativ zu der soeben beschriebenen Ausgestaltungsvariante kann nach der Erfindung auch vorgesehen sein, daß der Docht quer zu den Feldlinien des elektrischen Ursprungsfeldes mit dem flüssigen Stoff beschickt wird, und die schlitzförmigen Ausnehmungen im Wesentlichen in Richtung der Beschickung weisen. Diese Anordnung hat den Vorteil, daß die schlitzförmigen Ausnehmungen die kapillare Flüssigkeitsströmung am wenigsten behindern, erfordert allerdings neben den elektrischen Kontaktstützpunkten einen weiteren Stützpunkt für die kapillare Kontaktierung des Dochts.

Zweckmäßige und vorteilhafte Ausführungsbeispiele der Erfindung sind in den Zeichnungen dargestellt und werden in der nachfolgenden Beschreibung näher erläutert.

Es zeigen:
Fig.1 einen flächigen Verdampfer ohne schlitzförmige Ausnehmungen;
Fig. 2 einen erfindungsgemäßen flächigen Verdampfer mit schlitzförmigen Ausnehmungen;
Fig. 3 die Leistungsdichte-Verteilung des flächigen Verdampfers nach Fig. 2;
Fig. 4 die Temperaturverteilung im Querschnitt A-A gemäß Fig. 3 während der impulsartigen Aufheizung und Verdampfung;
Fig. 5 die Stofffreisetzung des flächigen Verdampfers nach Fig. 2 während der impulsartigen Verdampfung;
Fig. 6 die Stofffreisetzung des flächigen Verdampfers nach Fig. 1 während der impulsartigen Verdampfung;
Fig. 7 eine Verdampfer-Anordnung mit selbsttätiger Flüssigkeitsversorgung;
Fig. 8 und 9 eine weitere Verdampfer-Anordnung mit selbsttätiger Flüssigkeitsversorgung.

Tabelle 1 zeigt die Material-Spezifikationen eines beispielhaften erfindungsgemäßen flächigen Verdampfers. Demnach besteht der flächige Verdampfer aus sechs Lagen: aus einer Metallfolie und fünf darauf aufgesinterten Metalldrahtgeweben. Das Metall besteht im vorliegenden Beispiel aus der Heizleiterlegierung NiCr8020, DIN-Werkstoff-Nummer 2.4869. Es könnten selbstverständlich auch andere Heizleiterlegierungen Verwendung finden. Heizleiterlegierungen können als Vormaterial von der Firma ThyssenKrupp VDM GmbH, www.thyssenkruppvdm.de bezogen werden und anschließend zu Folien, Feinstdrähten und Drahtgeweben weiterverarbeitet werden. Die Firma ThyssenKrupp VDM GmbH führt den zuvor genannten Werkstoff NiCr8020, DIN-Werkstoff-Nummer 2.4869, unter der Handelsbezeichnung "Cronix 80", wobei auch eine sehr reine Spezifikation mit einem Kohlenstoffgehalt <0,02% erschmolzen werden kann. Die Firma Record Metall-Folien GmbH, www.recordmetall.de ist in Kooperation mit ihren Vorlieferanten in der Lage, aus dem vorzugsweise als Band vorliegenden Vormaterial Metallfolien ab einer Dicke von 5µm herzustellen. Die Firma J. G. Dahmen & Co. GmbH & Co. KG, www.dahmen-draht.de ist in der Lage, aus dem vorzugsweise als Draht vorliegenden Vormaterial Feinstdrähte ab einer Dicke von 18µm zu ziehen. Die Feinstdrähte können anschließend von Drahtwebereien, zum Beispiel von den Firmen Haver & Boecker, www.haverboecker.com oder Spörl KG, www.spoerl.de routinemäßig zu Drahtgeweben verwoben werden.

Die Metallfolie und die Drahtgewebelagen sind durch eine Sinterung miteinander verbunden. Die Sinterung erfolgt vorzugsweise im Vakuum oder unter einer Wasserstoff-Schutzatmosphäre. Solche Sinterungen zählen zum Stand der Technik und werden beispielsweise von der Firma GKN Sinter Metals Filters GmbH, www.gkn-filters.com sowie von der Firma Spörl KG, www.spoerl.de routinemäßig durchgeführt. Die Sinterung erfolgt vorteilhafterweise in Form eines Vielfachnutzens; das heißt, daß nicht einzelne flächige Verdampfer gesintert werden, sondern größere flächige Nutzen beispielsweise im Format 200x200mm. Die einzelnen flächigen Verdampfer werden nach der Sinterung durch Laserschneiden oder Stanzen aus dem Vielfachnutzen gewonnen.

Anstatt Heizleiterlegierungen könnten alternativ auch Edelstähle, z.B. AISI 304L oder AISI 316L, als Material für den erfindungsgemäßen flächigen Verdampfer Verwendung finden. Diese haben gegenüber NiCr8020 erhebliche Kostenvorteile, weisen allerdings im Vergleich zu NiCr8020 einen doch deutlich niedrigeren elektrischen Widerstand auf. Zur Erhöhung des elektrischen Widerstandes können die aus Edelstahl hergestellten flächigen Verdampfer oder deren Produktionsvorstufe - der Vielfachnutzen - optional geätzt werden. Durch die Ätzung wird Material gleichmäßig abgetragen. Die Ätzung kann vorteilhafterweise in einem wässrigen Beizbad bestehend aus 50% Salpetersäure und 13% Flussäure erfolgen. Eine solche Badbeize kann unter der Bezeichnung "Avesta 302" von der **Firma Avesta Finishing Chemicals, www.avestafinishing.com, bezogen werden. Ein** günstiger Nebeneffekt der Ätzung ist, daß sich durch sie auch die Porosität des flächigen Verdampfers etwas erhöht, wodurch der Verdampfer noch aufnahmefähiger für den zu verdampfenden Stoff wird. Der zusätzliche Verfahrensschritt der Ätzung gleicht allerdings die ursprünglichen Kostenvorteile gegenüber Heizleiterlegierungen zumindest teilweise wieder aus.

Eine interessante Option besteht auch darin, Heizleiterlegierungen und Edelstähle miteinander zu kombinieren, wobei vorzugsweise die massiveren Lagen aus einer Heizleiterlegierung und die feineren Lagen aus Edelstahl bestehen. Im konkreten Ausführungsbeispiel nach Tabelle 1 könnten nach dieser Option beispielsweise die äußeren drei Gewebelagen, also die 3. bis 5. Gewebelage auch aus Edelstahl bestehen.

**Tabelle1: Material-Spezifikationen**

| | | |
|---|---|---|
| Material: | NiCr8020 | DIN 2.4869, "Cronix80" |
| Metallfolien-Dicke: | 5µm | alternativ 10µm |
| 1. Drahtgewebelage: | 90x36µm | Maschenweite x Drahtdurchmesser |
| 2. Drahtgewebelage: | 71x30µm | Maschenweite x Drahtdurchmesser |
| 3. Drahtgewebelage: | 56x20µm | Maschenweite x Drahtdurchmesser |
| 4. Drahtgewebelage: | 65x20µm | Maschenweite x Drahtdurchmesser |
| 5. Drahtgewebelage: | 45x18µm | Maschenweite x Drahtdurchmesser |
| Dicke: | 222µm | nach dem Sintern |
| Porosität: | 70% | |
| Spezifischer elektrischer Widerstand: | 8,4 [mOhm mm] | |
| Wärmeleitfähigkeit: | 2,1-2,7 *) [W/mK] | |

| | | |
|---|---|---|
| *) im Temperaturbereich 20°C bis 400°C | | |

Bei nach Tabelle 1 aufgebauten flächigen Verdampfern wird die Porenstruktur durch die miteinander versinterten Drahtgewebelagen gebildet. Sowohl die Drahtgewebelagen als auch die Metallfolie leisten einen Beitrag zum resultierenden elektrischen Heizwiderstand. Die in Tabelle 1 angeführten Werte für den spezifischen elektrischen Widerstand und für die Wärmeleitfähigkeit gelten selbstverständlich in Flächenrichtung. Die Werte nehmen mit zunehmender Porosität überproportional ab.

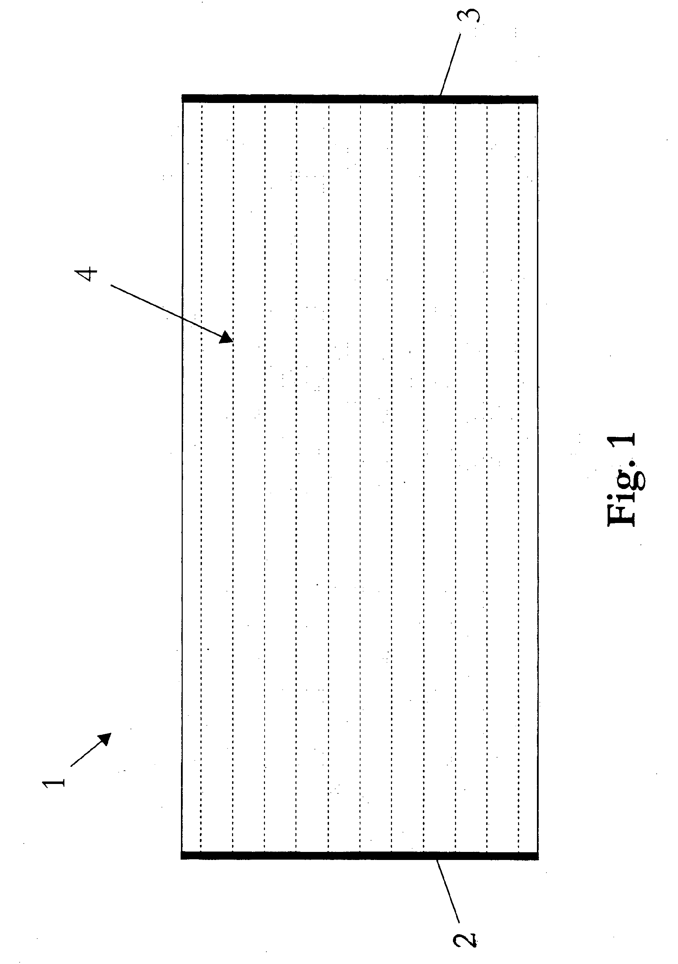

Fig. 1 zeigt exemplarisch eine rechteckige Anordnung eines flächigen Verdampfers 1. Die Abmessungen des Verdampfers betragen: Länge=12mm, Breite=5,6mm. Der Aufbau des Verdampfers entspricht jenem nach Tabelle 1. Der Verdampfer bzw. dessen elektrisches Widerstandsmaterial ist an seinen schmalen Enden elektrisch kontaktiert, bildet also an diesen Enden zwei Pole 2, 3 aus, über welche der Heizstrom zu- und wieder abgeleitet wird. Der Heizstrom fließt, sobald an den Polen 2, 3 eine Spannung angelegt wird. Die in Fig. 1 dargestellte Anordnung kann auch nur einen beheizten oder beheizbaren Ausschnitt eines flächigen Verdampfers 1 darstellen. Das Widerstandsmaterial samt Porenstruktur kann sich nämlich über die Pole 2, 3 hinaus fortsetzen. Beispielsweise kann der flächige Verdampfer 1 jenseits der Pole 2, 3 vorzugsweise folienseitig mittels eines elektrisch leitenden Klebstoffes oder durch eine Schweißverbindung flächig kontaktiert sein, wodurch gleichzeitig auch eine mechanische Befestigung des Verdampfers bewirkt wird. Die linienförmigen Pole 2, 3 würden in diesem Fall die äußere Berandung der flächigen Kontaktierung darstellen. Wie man einfach nachrechnen kann, beträgt der resultierende Heizwiderstand des in Fig. 1 dargestellten flächigen Verdampfers 81mOhm. Fig. 1 zeigt ferner die Feldlinien 4 des sich zwischen den Polen 2, 3 ausbildenden elektrischen Ursprungsfeldes, wenn an den Polen 2, 3 eine Spannung anliegt. Demnach verlaufen die Feldlinien geradlinig und verbinden die beiden Pole auf kürzestem Weg. Die dargestellte Verdampfer-Anordnung weist eine absolut homogene Strom- und Leistungsdichte-Verteilung auf.

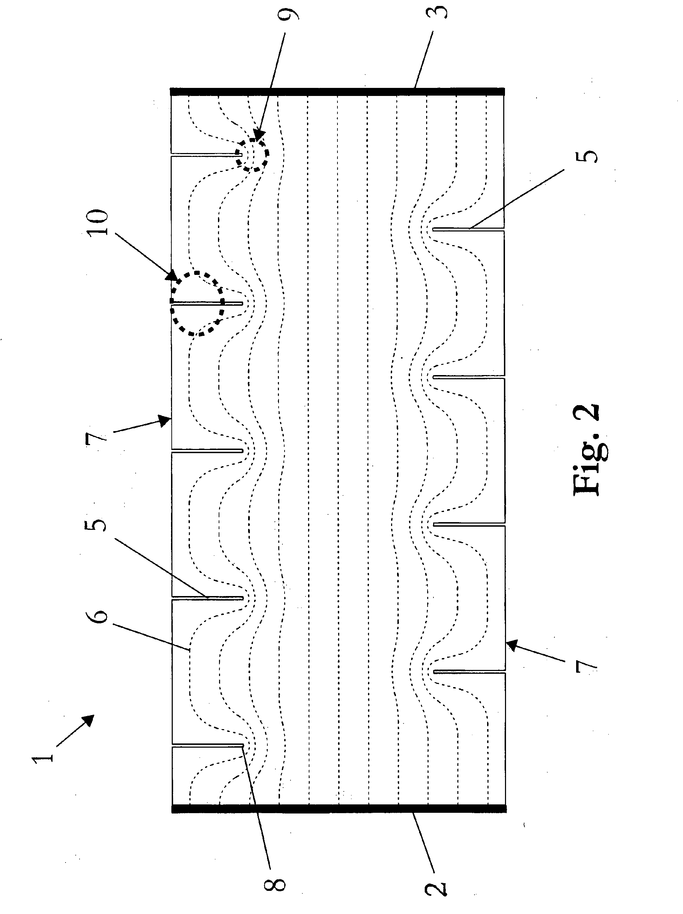

Fig. 2 zeigt denselben flächigen Verdampfer wie Fig. 1, jedoch nun mit erfindungsgemäßen schlitzförmigen Ausnehmungen 5. Die Ausnehmungen 5 sind zu den Feldlinien 4 des elektrischen Ursprungsfeldes (siehe Fig. 1) orthogonal ausgerichtet, wodurch dieselbigen Feldlinien eingeschnürt werden. Die eingeschnürten Feldlinien sind in Fig. 2 mit dem Bezugszeichen 6 gekennzeichnet. Insgesamt sind neun Schlitze 5 vorgesehen. Die Schlitze sind im konkreten Ausführungsbeispiel als Einschnitte ausgebildet. Die Länge der Einschnitte beträgt 1,2mm. Die Einschnitte 5 gehen von gegenüberliegenden Rändern 7 des flächigen Verdampfers 1 aus. Die Einschnitte können vorteilhafterweise mittels Nd:YAG-Laser gesetzt werden. Mit diesem Schneidverfahren können Einschnitte mit einer Breite ab etwa 50µm ausgeführt werden. Die Vorteile solch feiner Einschnitte wurden schon früher dargestellt. Durch die Einschnitte 5 steigt der resultierende Heizwiderstand des flächigen Verdampfers auf 110mOhm an, das ist eine Steigerung um etwa 35%.

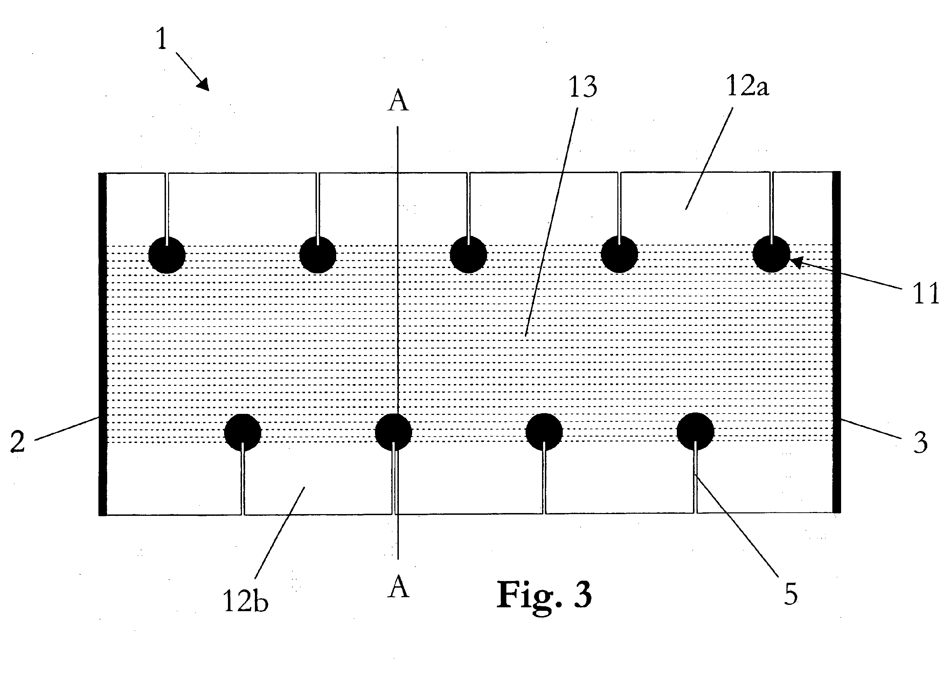

Die Einschnürung der elektrischen Feldlinien 6 hat eine ungleichmäßige Verteilung der elektrischen Feldstärke zur Folge. Konkret treten in den an die Enden 8 der Einschnitte 5 unmittelbar angrenzenden Zonen 9, wo die Feldlinien am stärksten eingeschnürt werden, fast punktuell besonders hohe Feldstärken auf, während in den an die Einschnitte 5 längsseitig angrenzenden Zonen 10 die Feldstärke vergleichsweise kleine Werte aufweist. Da die Feldstärke in die Berechnung der elektrischen Leistungsdichte quadratisch eingeht, kann erwartet werden, daß die elektrische Leistungsdichte noch wesentlich ausgeprägter schwankt, was, wie Fig. 3 zeigt, auch tatsächlich der Fall ist. Grob betrachtet können drei Zonen unterschieden werden: zum einen an die Enden der Einschnitte 5 unmittelbar angrenzende und wiederum beinahe punktuell in Erscheinung tretende Zonen 11 höchster Leistungsdichte (schwarz dargestellt). Zum anderen zwei über die Längsseiten des flächigen Verdampfers 1 sich erstreckende Randzonen 12a und 12b geringster Leistungsdichte, welche sich in Breitenrichtung etwa bis zu den Enden der Einschnitte 5 ausdehnen (weiß dargestellt). Und schließlich eine mittlere Zone 13 mittlerer Leistungsdichte, welche sich im Wesentlichen zwischen den Randzonen 12a und 12b ausdehnt (punktiert dargestellt). Diese ausgeprägt inhomogene Leistungsdichte-Verteilung führt zusammen mit der schlechten Wärmeleitfähigkeit der Porenstruktur dazu, daß sich während der impulsartigen Aufheizung in der Porenstruktur und damit auch im zu verdampfenden Stoff steile Temperaturgradienten ausbilden. Das nun folgende Rechenbeispiel und die Ergebnisse daraus basieren auf der Auswertung der Wärmeleitgleichung für die in Fig. 2 abgebildete Geometrie und auf den Voraussetzungen gemäß der Tabellen 2 und 3.

Tabelle 2 beschreibt die Zusammensetzung des zu verdampfenden Stoffes. Demnach besteht der Stoff im konkreten Beispiel aus einer mittels Ethanol und Wasser hochverdünnten Nikotinlösung. Als Aerosolbildner findet Glycerol Verwendung. Ferner sind eine Reihe von organischen Säuren zur Protonierung des Nikotins beigesetzt. Die Poren der Porenstruktur seien vollständig mit der Nikotinlösung gefüllt, wonach in der Porenstruktur insgesamt 10,4µL Nikotinlösung gespeichert sind. Die Stofffreisetzung im Zuge der Verdampfung wird im Wesentlichen durch das Dreistoffsystem Ethanol-Wasser-Glycerol bestimmt. Die Aufgabe, die in Tabelle 2 angeführten Inhaltsstoffe möglichst synchron freizusetzen, ist schon erfüllt, wenn es gelingt, die beiden Hauptstoffgruppen, das Lösungsmittel (Ethanol und Wasser) einerseits und den Aerosolbildner (Glycerol) andererseits, möglichst synchron freizusetzen. Daß diese Aufgabe nicht einfach ist, zeigen schon alleine die erheblich unterschiedlichen Stoffeigenschaften dieser Stoffgruppen - siehe Tabelle 2a.

**Tabelle 2: Exemplarische Arzneimittel-Zubereitung auf Basis Nikotin**

| Stoff | CAS-Nummer | Massen-% |
|---|---|---|
| Ethanol | 64-17-5 | 67,13 |
| Wasser | 7732-18-5 | 17,72 |
| Glycerol | 56-81-5 | 11,08 |
| Nikotin | 54-11-5 | 1,33 |
| Milchsäure | 50-21-5 | 0,11 |
| Bernsteinsäure | 110-15-6 | 0,18 |
| Lävulinsäure | 123-76-2 | 0,40 |
| Benzoesäure | 65-85-0 | 0,07 |
| Essigsäure | 64-19-7 | 1,30 |
| Ameisensäure | 64-18-6 | 0,43 |
| Solanon | 1937-54-8 | 0,07 |
| Tabakaromaöle | | 0,18 |
| | Summe: | 100,00 |

**Tabelle 2a: Stoffeigenschaften Ethanol-Wasser-Glycerol**

| | Siedetemperatur | Dampfdruck bei 20°C |
|---|---|---|
| Ethanol | 78°C | 5800 Pa |
| Wasser | 100°C | 2340 Pa |
| Glycerol | 290°C | <0,1 Pa |

Bei der Lösung der Wärmeleitgleichung geht die in Fig. 3 dargestellte elektrische Leistungsdichte als Wärmequelle ein. Für die Berechnung der Leistungsdichte wurde eine an den Polen 2, 3 anliegende Gleichspannung in Höhe von 1,5V zugrundegelegt, wobei die Stromzufuhr gemäß Tabelle 3 mit einem variablen Aussteuerungsgrad (Duty Cycle) erfolgt. Auf diese Weise kann die Energiezufuhr während der Dauer der impulsartigen Aufheizung und Verdampfung beliebig gesteuert werden. Die resultierende Nutzspannung ist also ein Rechteck-Signal konstanter Amplitude aber mit einem variablen Tastverhältnis. Ferner wurde die Temperatur an den Polen 2, 3 konstant auf Umgebungstemperatur (20°C) gesetzt, was etwa auch zutrifft, wenn der flächige Verdampfer 1 an den Polen gleichzeitig auch befestigt ist.

**Tabelle 3: Bestromung - Aussteuerungsgrad [%]**

| Zeit [s] | [%] |
|---|---|
| 0,00 - 0,52 | 100 |
| 0,52 - 0,55 | 80 |
| 0,55 - 0,60 | 60 |
| 0,60 - 0,70 | 40 |
| 0,70 - 1,40 | 35 |
| 1,40 - 1,50 | 25 |
| 1,50 - 1,55 | 15 |
| 1,55 - 1,60 | 10 |
| 1,60 - 2,00 | 0 |

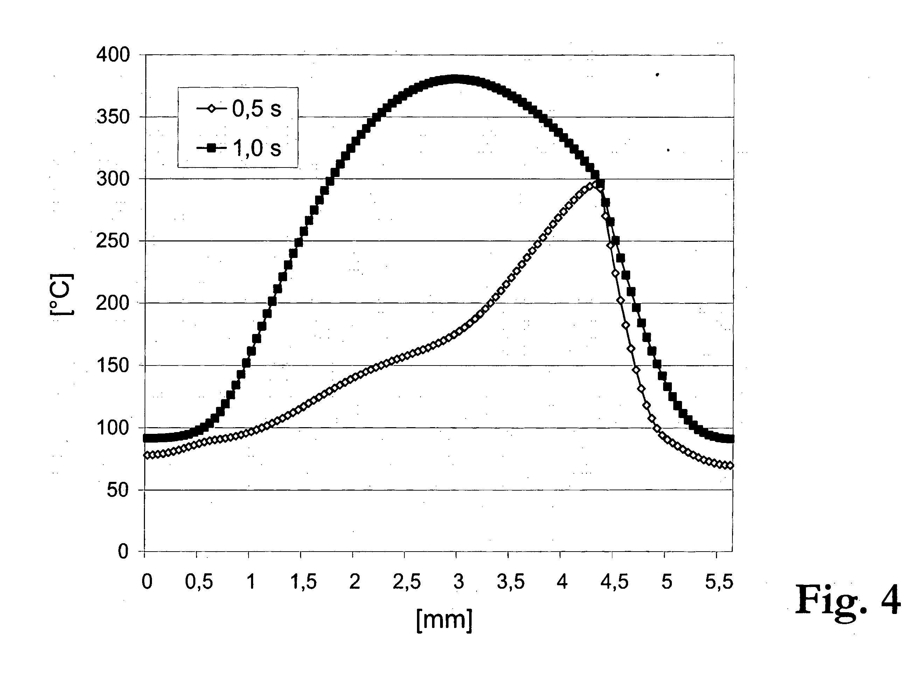

Fig. 4 zeigt als ein erstes Ergebnis der Auswertung die Temperaturverteilung im Querschnitt A-A gemäß Fig. 3 und zwar 0,5 bzw. 1,0 Sekunden nach Beginn der impulsartigen Aufheizung. Demnach treten in der Porenstruktur und damit auch in der Nikotinlösung Temperaturgradienten in der Größenordnung von 200°C/mm, teilweise sogar bis 350°C/mm auf. Diese Werte reichen durchaus bereits an die in einer glimmenden Zigarette auftretenden Temperaturgradienten heran.

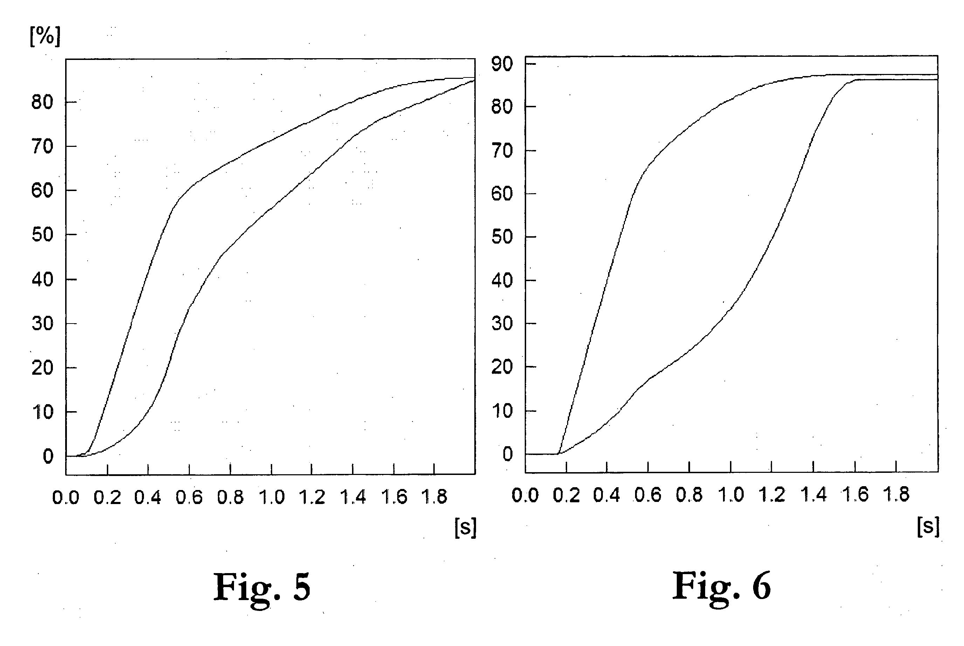

Fig. 5 zeigt als ein weiteres Ergebnis der Simulation die während der impulsartigen Verdampfung kumulativ freigesetzten Mengen an Lösungmittel (Ethanol und Wasser) und Aerosolbildner (Glycerol). Die Werte sind prozentual und beziehen sich auf die ursprünglich in der Porenstruktur gespeicherten Ausgangsmengen. Bei der Berechnung wurde angenommen, daß die Freisetzung der Einzelkomponenten des Dreistoffsystems Ethanol-Wasser-Glycerol nur temperaturabhängig ist, und diese Abhängigkeit in guter Näherung durch ein Exponentialgesetz erfaßt werden kann. Die obere Kurve kennzeichnet das Lösungsmittel, die untere Kurve den Aerosolbildner. Fig. 6 zeigt im direkten Vergleich dazu die Stofffreisetzung bei Verwendung eines flächigen Verdampfers, wie in Fig. 1 abgebildet, also ohne Schlitze, wobei die an den Polen 2, 3 anliegende Spannung wegen des nun geringeren Heizwiderstandes auf 1,3V reduziert wurde (gleiche Leistungsdichte). Man sieht auf Anhieb, wie erheblich der Einfluß der schlitzförmigen Ausnehmungen bzw. Einschnitte 5 auf die Dynamik der Freisetzung ist. Die Stofffreisetzung nach Fig. 5 verläuft ausgesprochen synchron, wogegen das Lösungsmittel und der Aerosolbildner im Beispiel nach Fig. 6 beinahe gegenläufig freigesetzt werden. Es kann daraus der Schluß gezogen werden, daß der geschlitzte flächige Verdampfer 1 nach Fig. 2 für eine systemische Wirkstoffzufuhr über die Lungenalveolen, im konkreten Beispiel für eine systemische Nikotinverabreichung, wesentlich besser geeignet erscheint als Verdampfer ohne solche Schlitze.

Die Schlitzlänge ist auf die spezifischen Materialeigenschaften des flächigen Verdampfers, insbesondere auf die Wärmeleitfähigkeit der Porenstruktur abzustimmen, wobei hier letztlich ein Kompromiß gefunden werden muß: allzu lange Schlitze bzw. Einschnitte führen dazu, daß sich lokal Zonen ohne jegliche, oder ohne wesentliche Verdampfung ausbilden. Der zu verdampfende Stoff wird in solchen Zonen lediglich leicht erwärmt. Solche Zonen können auch als Totzonen bezeichnet werden, weil sie zur Verdampfung keinen, oder keinen nennenswerten **Beitrag leisten. Wird die Schlitzlänge hingegen allzu kurz gewählt kann das** Potenzial des Schlitzes, steile Temperaturgradienten auszubilden, nicht im vollen Umfang ausgeschöpft werden.

Die Versorgung des flächigen Verdampfers 1 nach Fig. 2 mit elektrischem Strom kann mittels eines wiederaufladbaren Akkus erfolgen. Nach dem heutigen Stand der Technik bieten sich zu diesem Zweck insbesondere Lithium-Ionen- sowie Lithium-Polymer-Zellen an. Diese Zelltypen stellen gegenwärtig die höchsten Energiedichten und -ströme zur Verfügung und werden seit längerem vielfältig eingesetzt, wobei an erster Stelle die breite Verwendung in Mobiltelefonen zu nennen ist. Schaltet man zwei flächige Verdampfer 1 nach Fig. 2 elektrisch in Reihe, und erfolgt die Energiezufuhr auf Basis einer einzelnen Lithium-Polymer-Zelle mit einer Nenn- oder Leerlaufspannung von 3,7V und einer Nutzspannung unter Last von ca. 3V, dann errechnet sich auf Basis des Ohmschen Gesetzes der Strom, welcher durch die flächigen Verdampfer fließt, zu 13,6A. Diese Stromstärke kann aus heutigen Lithium-Polymer-Zellen problemlos bezogen werden. Als Beispiel sei der Zelltyp SLPB533459H4 des Herstellers Kokam Co., Ltd, www.kokam.com, angeführt. Die Abmessungen der Zelle betragen: 58,5 x 33,5 x 5mm (L x B x H) bei einem Gewicht von 19,5g. Die Zelle verfügt über eine Kapazität von 740mAh und vermag, einen Strom von 14,8A kontinuierlich zu liefern. Legt man eine impulsartige Bestromung nach Tabelle 3 zugrunde, dann ziehen die beiden in Serie geschalteten Verdampfer pro impulsartige Verdampfung eine Strommenge von etwa 4,4mAh. Demnach wären mit der zuvor genannten Kokam-Zelle theoretisch bis zu 168 Verdampfungszyklen bzw. Inhalationen möglich. Real wird dieser Wert wegen der relativ hohen Stromstärke und der impulsartigen Art des Strombezuges wohl nicht ganz erreicht werden können. Die genannte Zelle kann allerdings sehr schnell wieder aufgeladen werden.

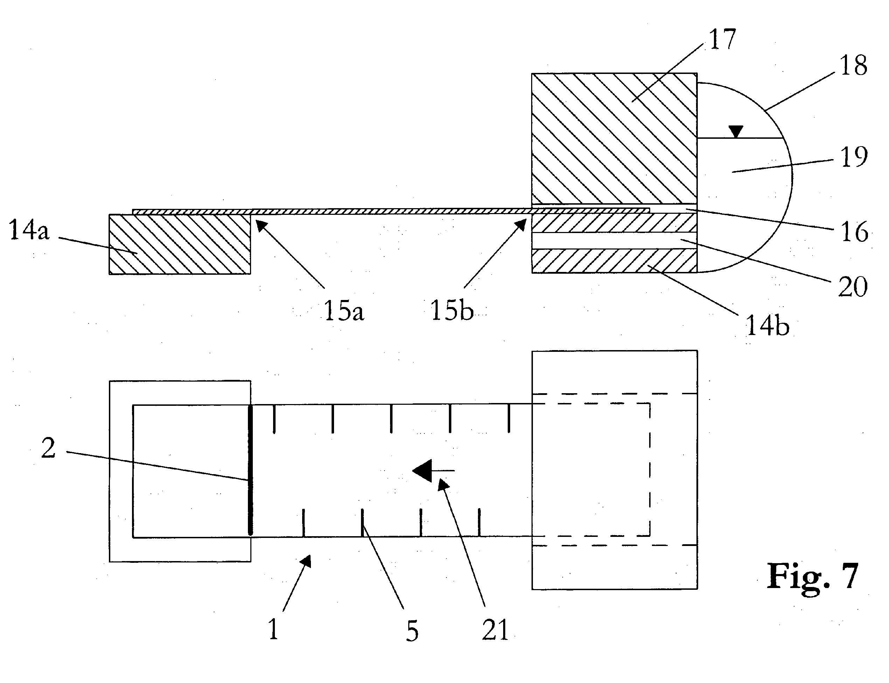

Fig. 7 zeigt eine Verdampfer-Anordnung innerhalb einer Inhalatorkomponente, wobei der flächige Verdampfer 1 von der Geometrie und vom Aufbau her identisch ist mit der Anordnung nach Fig. 2. Der flächige Verdampfer 1 lagert mit zwei Endabschnitten vorzugsweise folienseitig auf zwei elektrisch leitenden plattenförmigen Kontaktelementen 14a und 14b, auf deren Oberfläche er auch gleichzeitig elektrisch kontaktiert ist. Die Kontaktierung kann beispielsweise durch eine flächige Klebeverbindung mittels eines leitfähigen Klebstoffes, z.B. der Firma Epoxy Technology, www.epotek.com, erfolgen. Der Heizstrom fließt, sobald an den **Kontakteleme**nten **14a** und **14b eine Spannung anlegt. Da die elektrische** Leitfähigkeit der Kontaktelemente 14a und 14b um ein Vielfaches größer ist als die Leitfähigkeit des flächigen Verdampfers 1, können die äußeren Berandungen 15a und 15b der Kontaktelemente, das sind jene Stellen, an welchen der flächige Verdampfer 1 die Kontaktelemente 14a und 14b erstmalig berührt, als Pole 2, 3 aufgefaßt werden. Der Pol 2 ist in Fig. 7 durch eine dicke Linie angedeutet. Der flächige Verdampfer 1 ragt mit einem Endabschnitt in einen Kapillarspalt 16. Der Kapillarspalt 16 wird durch das Kontaktelement 14b und ein auf dieses flächig aufgesetztes Oberteil 17 gebildet, indem im Oberteil 17 eine entprechende Ausnehmung, z.B. Einfräsung, eingearbeitet ist. Der Kapillarspalt 16 kommuniziert mit einem Flüssigkeitsspeicher 18 (in Fig. 7 symbolisch dargestellt). Der Flüssigkeitsspeicher 18 beinhaltet den zu verdampfenden Stoff bzw. die zu verdampfende Flüssigkeit 19. Die Flüssigkeit 19 könnte beispielsweise aus einer verdünnten Arzneimittel-Zubereitung bestehen; eine solche Zubereitung wurde exemplarisch bereits in Tabelle 2 beschrieben. Ferner befindet sich im Kontaktelement 14b eine Belüftungsbohrung 20, welche ebenfalls mit dem Flüssigkeitsspeicher 18 kommuniziert. Die Anordnung hat folgende Wirkung: der Kapillarspalt 16 zieht durch die in ihm wirkenden Kapillarkräfte Flüssigkeit aus dem Flüssigkeitsspeicher 18, wodurch sich der Kapillarspalt 16 mit Flüssigkeit 19 füllt. Die Flüssigkeit 19 kommt im Kapillarspalt 16 mit der Porenstruktur des flächigen Verdampfers 1 in Kontakt. Die Porenstruktur wird von der Flüssigkeit 19 benetzt und wirkt ihrerseits wie ein Docht, wodurch sich die gesamte Porenstruktur des flächigen Verdampfers 1 selbsttätig mit der Flüssigkeit 19 füllt. Zum Ausgleich der entnommenen Flüssigkeitsmenge strömt Luft aus der Umgebung über die Belüftungsbohrung 20 in den Flüssigkeitsspeicher 18 nach. Nach einer jeden impulsartigen Aufheizung und Verdampfung der Flüssigkeit 19 wiederholt sich der Befüllungsvorgang von neuem. Die Porenstruktur wirkt in diesem Fall also multifunktionell: erstens unterstützt die Porenstruktur dank ihrer geringen Wärmeleitfähigkeit die Ausbildung von steilen Temperaturgradienten in der zu verdampfenden Flüssigkeit 19; zweitens fixiert sie die Flüssigkeit 19 während der Verdampfung in ihren Poren, wodurch ein Stoffaustausch in Flächenrichtung unterbunden wird und bereits ausgebildete Temperaturgradienten nicht gestört werden; und drittens füllt sie sich nach einer impulsartigen Verdampfung durch die in ihr wirkenden Kapillarkräfte von neuem selbsttätig mit der zu verdampfenden Flüssigkeit 19.

Die Versorgung der Kapillarstruktur mit der zu verdampfenden Flüssigkeit 19 erfolgt im Ausführungsbeispiel nach Fig. 7 in Richtung der Feldlinien 4 des elektrischen Ursprungsfeldes (vgl. Fig. 1). Die schlitzförmigen Ausnehmungen bzw. Einschnitte 5 sind hintereinander gestaffelt in zwei zu den Feldlinien im Wesentlichen parallel ausgerichteten Reihen angeordnet. Hierdurch wird eine dem Docht vorbehaltene zentrale Hauptversorgungsader 21 geschaffen, welche weitgehend frei von schlitzförmigen Ausnehmungen ist, und über welche die Porenstruktur weitgehend ungehindert mit der Flüssigkeit 19 versorgt werden kann (in Fig. 7 durch einen Pfeil angedeutet).

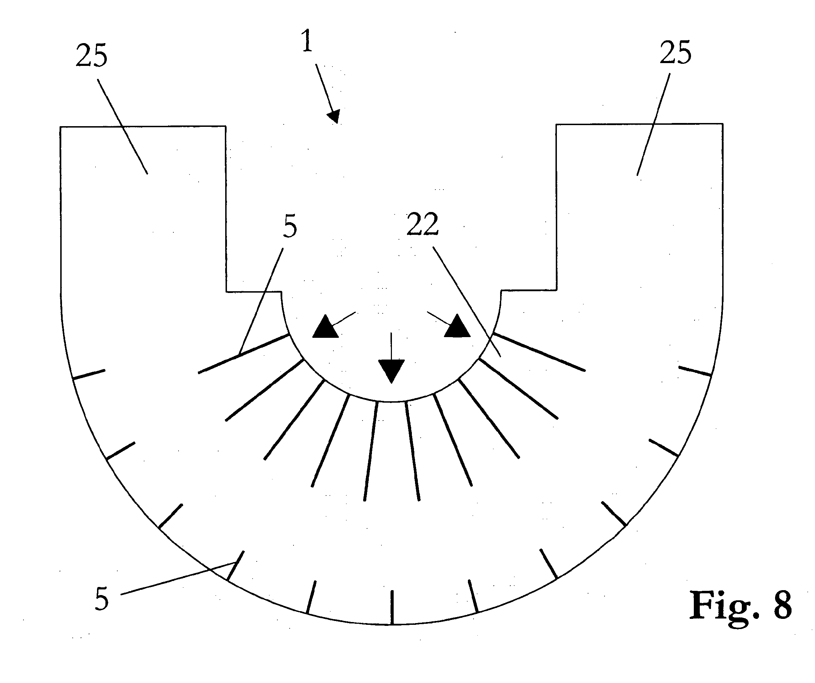

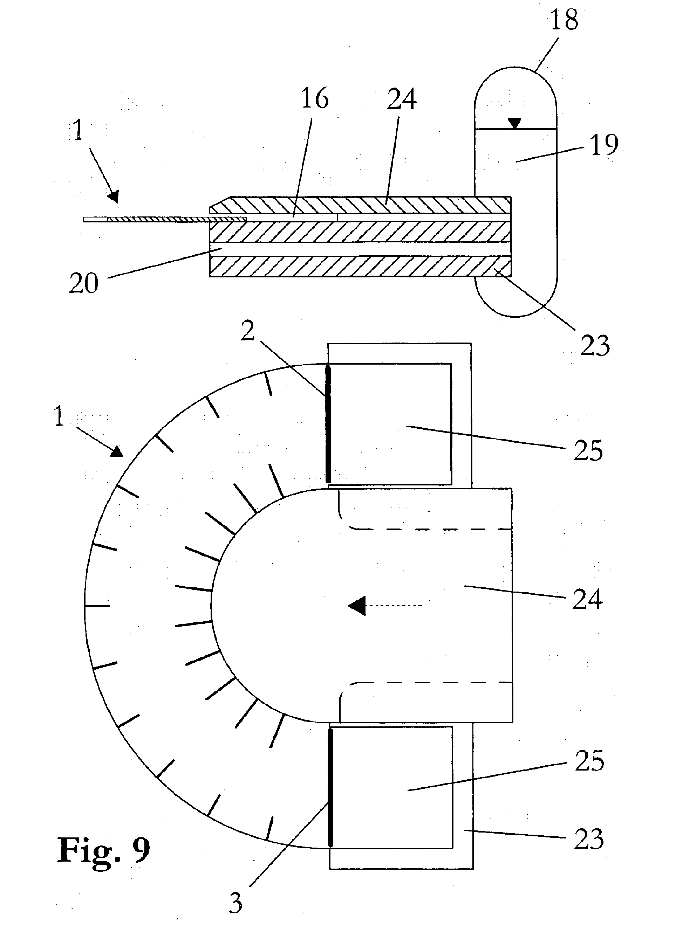

Fig. 8 und 9 zeigen eine ringförmige Verdampfer-Anordnung. Der flächige Verdampfer 1 sei wiederum, wie in Tabelle 1 dargestellt, aufgebaut und wird auch in diesem Ausführungsbeispiel wieder über einen Kapillarspalt 16 mit der zu verdampfenden Flüssigkeit 19 versorgt. Im Unterschied zu dem zuvor dargestellten Ausführungsbeispiel erfolgt die Beschickung der Kapillarstruktur hier quer zu den Feldlinien des elektrischen Ursprungsfeldes, und die schlitzförmigen Ausnehmungen bzw. Einschnitte 5 weisen in Richtung der Beschickung. Konkret erfolgt die Beschickung radial von innen nach außen (in Fig. 8 durch Pfeile angedeutet) und die Einschnitte 5 sind ebenfalls radial ausgerichtet. Diese Art der Beschickung hat den Vorteil, daß die kapillare Strömung im Docht durch die Einschnitte 5 nicht behindert wird. Es sind sowohl Einschnitte am inneren als auch am äußeren Umfang vorgesehen. Die Einschnitte am inneren Umfang bilden Zungen 22 aus, welche teilweise in den Kapillarspalt 16 hineinragen und auf diese Weise die kapillare Kopplung mit der Flüssigkeit 19 bewirken. Der Kapillarspalt 16 wird durch eine Leiterplatte 23 und eine auf diese flächig aufgesetzte Abdeckplatte 24 gebildet, indem in der Abdeckplatte 24 eine entprechende Ausnehmung, z.B. Einfräsung, eingearbeitet ist. Der Kapillarspalt 16 bezieht die zu verdampfende Flüssigkeit 19 wieder aus einem Flüssigkeitsspeicher 18 (in Fig. 9 symbolisch dargestellt), und es ist auch wieder eine Belüftungsbohrung 20 für den Druckausgleich vorgesehen. Die elektrische Kontaktierung des flächigen Verdampfers 1 erfolgt über zwei Fortsätze 25, indem dieselbigen vorzugsweise folienseitig mittels eines leitfähigen Klebstoffes auf der Leiterplatte 23 flächig befestigt werden. Die Pole 2, 3 sind in Fig. 9 jeweils durch eine dicke Linie angedeutet.

Bei der Auswahl der Materialien für die den Kapillarspalt 16 ausbildenden Bauteile ist auf eine gute Benetzbarkeit derselbigen mit der zu verdampfenden Flüssigkeit 19 zu achten. Diese Bedingung gilt sowohl für die Anordnung nach Fig. 7 als auch für die Anordnung nach Fig. 9.

Der in Tabelle 1 exemplarisch dargestellte Aufbau eines erfindungsgemäßen flächigen Verdampfers basiert darauf, daß mehrere Metallgewebelagen auf einer Metallfolie aufgesintert sind. Bei Verdampfern dieser Bauart kann der gebildete Dampf nur auf der der Metallfolie abgewandten Seite aus der Porenstruktur austreten. Alternativ zu dieser Bauart können auch flächige Verdampfer mit beidseitig freiliegender Porenstruktur Verwendung finden, wobei es besonders günstig ist, wenn das Widerstandsheizelement komplett von der Porenstruktur ausgebildet wird. Flächige Verdampfer dieser Bauart ermöglichen besonders hohe Verdampfungsraten. Ein solcher Verdampfer kann beispielsweise aus einem elektrisch leitfähigen offenporigen Schaummaterial bestehen. Ein solches Schaummaterial kann in Edelstahl-Ausführung, z.B. AISI 316L, von der Firma Mitsubishi Materials Corporation, www.mmc.co.jp, bezogen werden. Hierbei wird von einem standardmäßigen Schaummaterial mit einer Dicke von 0,5mm, einem Porendurchmesser im Bereich 50-150µm und einer Porosität von zirka 90% ausgegangen. Das Material kann durch Walzen in der Dicke beliebig bis auf etwa 100µm reduziert werden. Das verdichtete Material kann anschließend optional noch gesintert werden. Durch die Verdichtung sinkt natürlich auch die Porosität, welche aber bei Bedarf im Zuge einer anschließenden Ätzbehandlung wieder gesteigert werden kann. Heizleiterlegierungen, insbesondere aus der Gruppe der NiCr-Legierungen, z.B. NiCr8020, DIN-Werkstoff-Nr. 2.4869, können ebenfalls zu einem solchen Schaummaterial verarbeitet werden. Der flächige Verdampfer kann aus einer einzigen Schaumlage oder aus mehreren, miteinander versinterten Schaumlagen bestehen. Zur Steigerung der Stabilität und Festigkeit des flächigen Verdampfers kann der Schaum optional auf eine dünne Trägerlage, beispielsweise auf ein Drahtgewebe, bestehend aus Edelstahl oder einer Heizleiterlegierung, aufgesintert werden. Das flächige Schaummaterial kann durch Laserschneiden in jede beliebige Form gebracht werden. Die erfindungsgemäßen schlitzförmigen Ausnehmungen bzw. Einschnitte werden vorzugsweise wieder mittels Nd:YAG-Laser in das Schaummaterial eingebracht.

In allen zuvor dargestellten Ausführungsbeispielen gingen die schlitzförmigen Ausnehmungen bzw. Einschnitte von Rändern des flächigen Verdampfers aus. Selbstverständlich ist die Erfindung nicht auf diese Anordnung beschränkt. Vielmehr können die schlitzförmigen Ausnehmungen bzw. Einschnitte auch beabstandet von den Rändern angeordnet sein. Derart angeordnete Schlitze haben eine doppelteinschnürende Wirkung, da in diesem Fall beide Schlitzenden die Feldlinien des elektrischen Ursprungsfeldes einengen.

### Bezugszeichenliste

- 1: flächiger Verdampfer
- 2: erster Pol
- 3: zweiter Pol
- 4: Feldlinien des elektrischen Ursprungsfeldes
- 5: schlitzförmige Ausnehmung; Einschnitt
- 6: eingeschnürte Feldlinien
- 7: Rand
- 8: Schlitzende
- 9: Zone hoher elektrischer Feldstärke
- 10: Zone niedriger elektrischer Feldstärke
- 11: Zone höchster Leistungsdichte
- 12: Randzone geringster Leistungsdichte
- 13: mittlere Zone mittlerer Leistungsdichte
- 14: plattenförmiges Kontaktelement
- 15: Berandung
- 16: Kapillarspalt
- 17: Oberteil
- 18: Flüssigkeitsspeicher
- 19: Flüssigkeit
- 20: Belüftungsbohrung
- 21: Hauptversorgungsader
- 22: Zunge
- 23: Leiterplatte
- 24: Abdeckplatte
- 25: Fortsatz

## Patentansprüche

1. Flächiger Verdampfer, umfassend ein flächiges elektrisches Widerstandsheizelement zur impulsartigen Aufheizung und Verdampfung eines auf der Heizelementfläche verteilten oder verteilbaren und inhalativ aufnehmbaren Stoffes mittels eines flächig durch das Widerstandsheizelement fließenden oder fließbaren elektrischen Heizstromes mit zumindest zwei elektrischen Kontaktierungen bzw. Polen (2, 3) zur Einleitung des Heizstromes in das Widerstandsheizelement, **dadurch gekennzeichnet, daß** das Widerstandsheizelement mindestens eine die Feldlinien (4) des sich zwischen den Polen (2, 3) ausbildenden bzw. ausbildbaren elektrischen Ursprungsfeldes einschnürende schlitzförmige Ausnehmung (5) aufweist und flächig mit einer den Stoff aufnehmenden oder aufnehmbaren offenzelligen Porenstruktur verbunden ist.

2. Flächiger Verdampfer **nach Anspruch 1, dadurch gekennzeichnet, daß** die Porenstruktur selbst aus einem elektrischen Widerstandsmaterial besteht, und die schlitzförmige Ausnehmung (5) auch die Porenstruktur durchsetzt.

3. Flächiger Verdampfer **nach Anspruch 2, dadurch gekennzeichnet, daß** das Widerstandsheizelement komplett von der Porenstruktur ausgebildet wird.

4. Flächiger Verdampfer **nach einem der Ansprüche 1-3, dadurch gekennzeichnet, daß** die schlitzförmige Ausnehmung (5) im Wesentlichen geradlinig verläuft und zu den von ihr eingeschnürten Feldlinien (4) des elektrischen Ursprungsfeldes zumindest annähernd orthogonal ausgerichtet ist.

5. Flächiger Verdampfer **nach einem der Ansprüche 1-4, dadurch gekennzeichnet, daß** die schlitzförmige Ausnehmung (5) aus einem Einschnitt besteht.

6. Flächiger Verdampfer **nach Anspruch 5, dadurch gekennzeichnet, daß** das Widerstandsheizelement und die Porenstruktur aus einem metallischen Widerstandsmaterial bestehen.

7. Flächiger Verdampfer **nach einem der Ansprüche 1-6, gekennzeichnet durch** mehrere schlitzförmige Ausnehmungen (5).

8. Flächiger Verdampfer **nach einem der Ansprüche 1-6, gekennzeichnet durch** eine Vielzahl von schlitzförmigen Ausnehmungen (5).

9. Flächiger Verdampfer **nach Anspruch 7 oder 8, dadurch gekennzeichnet, daß** die schlitzförmigen Ausnehmungen (5) ungleichmäßig dicht auf der Heizelementfläche verteilt sind.

10. Flächiger Verdampfer **nach Anspruch 7 oder 8, dadurch gekennzeichnet, daß** die Porenstruktur einen Docht ausbildet.

11. Inhalatorkomponente, umfassend mindestens einen flächigen Verdampfer (1) **nach einem der Ansprüche 1-10.**

12. Inhalatorkomponente, umfassend einen flächigen Verdampfer (1) **nach Anspruch 10** und eine mit dem Docht kapillar kommunizierende bzw. kommunizierbare Flüssigkeitsquelle (18) zur Versorgung des Dochts mit dem flüssigen Stoff (19), **dadurch gekennzeichnet, daß** der Docht in Richtung der Feldlinien (4) des elektrischen Ursprungsfeldes mit dem flüssigen Stoff (19) versorgt wird, und die schlitzförmigen Ausnehmungen (5) hintereinander gestaffelt in einer zu den Feldlinien (4) im Wesentlichen parallel ausgerichteten Reihe angeordnet sind.

13. Inhalatorkomponente, umfassend einen flächigen Verdampfer (1) **nach Anspruch 10** und eine mit dem Docht kapillar kommunizierende bzw. kommunizierbare Flüssigkeitsquelle (18) zur Versorgung des Dochts mit dem **flüssigen Stoff (19), dadurch gekennzeichnete daß der Docht quer zu den** Feldlinien (4) des elektrischen Ursprungsfeldes mit dem flüssigen Stoff (19) beschickt wird, und die schlitzförmigen Ausnehmungen (5) im Wesentlichen in Richtung der Beschickung weisen.

## Claims

1. Planar vaporizer, comprising a planar electrical resistance heating element for the pulsed heating and vaporization of a substance, which is distributed or can be distributed on the heating element surface and can be taken up by inhalation, by means of an electric heating current that flows or can flow in a planar manner through the resistance heating element, with at least two electrical contacts and/or poles (2, 3) for introducing the heating current into the resistance heating element, **characterized in that** the resistance heating element has at least one slot-shaped recess (5), constricting the lines of flux (4) of the original electric field that forms or can form between the poles (2, 3), and is bonded in a planar manner with an open-cell porous structure that takes up or can take up the substance.

2. Planar vaporizer according to Claim 1, **characterized in that** the porous structure itself consists of an electrically resistant material, and the slot-shaped recess (5) also passes through the porous structure.

3. Planar vaporizer according to Claim 2, **characterized in that** the resistance heating element is formed entirely by the porous structure.

4. Planar vaporizer according to one of Claims 1-3, **characterized in that** the slot-shaped recess (5) runs essentially in a straight line and is aligned at least approximately at right angles to the lines of flux (4) of the original electric field constricted by it.

5. Planar vaporizer according to one of Claims 1-4, **characterized in that** the slot-shaped recess (5) consists of a cut.

6. Planar vaporizer according to Claim 5, **characterized in that** the resistance heating element and the porous structure consist of a metallic resistance material.

7. Planar vaporizer according to one of Claims 1-6, **characterized by** a number of slot-shaped recesses (5).

8. Planar vaporizer according to one of Claims 1-6, **characterized by** a large number of slot-shaped recesses (5).

9. Planar vaporizer according to Claim 7 or 8, **characterized in that** the slot-shaped recesses (5) are distributed on the heating element surface unevenly closely.

10. Planar vaporizer according to Claim 7 or 8, **characterized in that** the porous structure forms a wick.

11. Inhaler component, comprising at least one planar vaporizer (1) according to one of Claims 1-10.

12. Inhaler component, comprising a planar vaporizer (1) according to Claim 10 and a source of liquid (18), communicating and/or capable of communicating by capillary action with the wick to supply the wick with the liquid substance (19), **characterized in that** the wick is supplied with the liquid substance (19) in the direction of the lines of flux (4) of the original electric field, and the slot-shaped recesses (5) are arranged staggered one behind the other in a row aligned essentially parallel to the lines of flux (4).

13. Inhaler component, comprising a planar vaporizer (1) according to Claim 10 and a source of liquid (18) communicating and/or capable of communicating by capillary action with the wick to supply the wick with the liquid substance (19), **characterized in that** the wick is charged with the liquid substance (19) transversely in relation to the lines of flux (4) of the original electric field and the slot-shaped recesses (5) point essentially in the direction of the charging.

## Revendications

1. Évaporateur plat, comprenant un élément chauffant résistif électrique plat pour le chauffage impulsionnel et l'évaporation d'une substance qui est répartie ou peut être répartie sur la surface de l'élément chauffant et pouvant être absorbée par inhalation au moyen d'un courant de chauffage électrique qui circule ou peut circuler à plat à travers l'élément chauffant résistif avec au moins deux contacts ou pôles (2, 3) électriques pour envoyer le courant de chauffage dans l'élément chauffant résistif, **caractérisé en ce que** l'élément chauffant résistif présente au moins un évidement (5) en forme de fente qui enserre les lignes de champ (4) du champ électrique originel qui se forme ou peut être formé entre les pôles (2, 3) et il est relié à plat avec une structure poreuse à alvéoles ouvertes qui accueille ou peut accueillir la substance.

2. Évaporateur plat selon la revendication 1, **caractérisé en ce que** la structure poreuse se compose elle-même d'un matériau résistif électrique et l'évidement (5) en forme de fente est également entremêlé avec la structure poreuse.

3. Évaporateur plat selon la revendication 2, **caractérisé en ce que** l'élément chauffant résistif est entièrement formé par la structure poreuse.

4. Évaporateur plat selon l'une des revendications 1 à 3, **caractérisé en ce que** l'évidement (5) en forme de fente s'étend sensiblement de manière rectiligne et est orienté au moins de manière approximativement orthogonale par rapport aux lignes de champ (4) du champ électrique originel qu'il enserre.

5. Évaporateur plat selon l'une des revendications 1 à 4, **caractérisé en ce que** l'évidement (5) en forme de fente se compose d'une entaille.

6. Évaporateur plat selon la revendication 5, **caractérisé en ce que** l'élément chauffant résistif et la structure poreuse se composent d'un matériau résistif métallique.

7. Évaporateur plat selon l'une des revendications 1 à 6, **caractérisé par** plusieurs évidements (5) en forme de fente.

8. Évaporateur plat selon l'une des revendications 1 à 6, **caractérisé par** une pluralité d'évidements (5) en forme de fente.

9. Évaporateur plat selon la revendication 7 ou 8, **caractérisé en ce que** les évidements (5) en forme de fente sont répartis sur la surface de l'élément chauffant avec une densité irrégulière.

10. Évaporateur plat selon la revendication 7 ou 8, **caractérisé en ce que** la structure poreuse forme une mèche.

11. Composant d'inhalateur, comprenant au moins un évaporateur plat (1) selon l'une des revendications 1 à 10.

12. Composant d'inhalateur, comprenant un évaporateur plat (1) selon la revendication 10 et une source de liquide (18) qui communique ou peut communiquer par effet capillaire avec la mèche pour alimenter la mèche avec la substance liquide (19), **caractérisé en ce que** la mèche est. alimentée avec la substance liquide (19) dans la direction des lignes de champ (4) du champ électrique originel et les évidements (5) en forme de fente sont échelonnés les uns derrière les autres en une rangée orientée sensiblement en parallèle avec les lignes de champ (4).

13. Composant d'inhalateur, comprenant un évaporateur plat (1) selon la revendication 10 et une source de liquide (18) qui communique ou peut communiquer par effet capillaire avec la mèche pour alimenter la mèche avec la substance liquide (19), **caractérisé en ce que** la mèche est chargée avec la substance liquide (19) transversalement aux lignes de champ (4) du champ électrique originel et les évidements (5) en forme de fente sont dirigés sensiblement dans la direction de la charge.
